# EUROPEAN PATENT SPECIFICATION

(11) **EP 2 277 852 B1**
(45) Date of publication and mention of the grant of the patent: **07.08.2013**
(21) Application number: 09178838.0
(22) Date of filing: 11.12.2009
(51) Int. Cl.: C07C 231/12, C07C 237/46

(54) **Synthesis of iodixanol in water**
Synthese von Iodixanol in Wasser
Synthèse d'iodixanol dans l'eau

(30) Priority: 21.07.2009 US 227094 P
(43) Date of publication of application: 26.01.2011
(73) Proprietor: GE Healthcare AS, 0401 Oslo (NO)
(72) Inventor: Brekke, Geir, N-0493, Oslo (NO); Rogne, Otto, N-0772, Oslo (NO); Kjernli, Trond, N-2322, Ridabu (NO); Svendsen, Terje, N-1385, Asker (NO)
(74) Representative: O'Brien, Dominic Paul

(56) References cited:
- EP-A1- 0 108 638
- WO-A1-98/23296
- WO-A1-2007/064220
- GB-A- 2 331 098

## Description

### TECHNICAL FIELD

This invention relates to the synthesis of iodixanol (1,3-bis(acetamido)-N,N'-bis[3,5-bis(2,3-dihydroxypropylaminocarbonyl)-2,4,6-triiodophenyl]-2-hydroxypropane), more specifically to the dimerisation of 5-acetamido-N,N'-bis(2,3-dihydroxypropyl)-2,4,6-triiodo-isophthalamide with water as solvent.

### BACKGROUND OF THE INVENTION

lodixanol is the non-proprietory name of the chemical drug substance of a non-ionic X-ray contrast agent marketed under the trade name Visipaque™. Visipaque™ is one of the most used agents in diagnostic X-ray procedures and is manufactured in large quantities.

The manufacture of such non-ionic X-ray contrast agents involves the production of the chemical drug substance (referred to as primary production) followed by formulation into the drug product (referred to as secondary production). Primary production of iodixanol involves a multi step chemical synthesis and a thorough purification process. For a commercial drug product it is important for the primary production to be efficient and economical and to provide a drug substance fulfilling the specifications, e.g. as expressed in the US Pharmacopea.

A number of methods are known for the preparation of iodixanol. These are all multi step chemical synthetic processes and the cost of the final formulated product thus mainly depends on these processes. It is therefore important to optimize the processes both for economic and environmental reasons.

In a preferred method for the preparation of iodixanol described in EP 108638 the final intermediate 5-acetamido-N,N'-bis(2,3-dihydroxypropyl)-2,4,6-triiodo-isophthalamide (hereinafter "Compound A") is reacted with a dimerisation agent such as epichlorohydrin, in a dimerisation step, to yield the drug substance, see Scheme I.

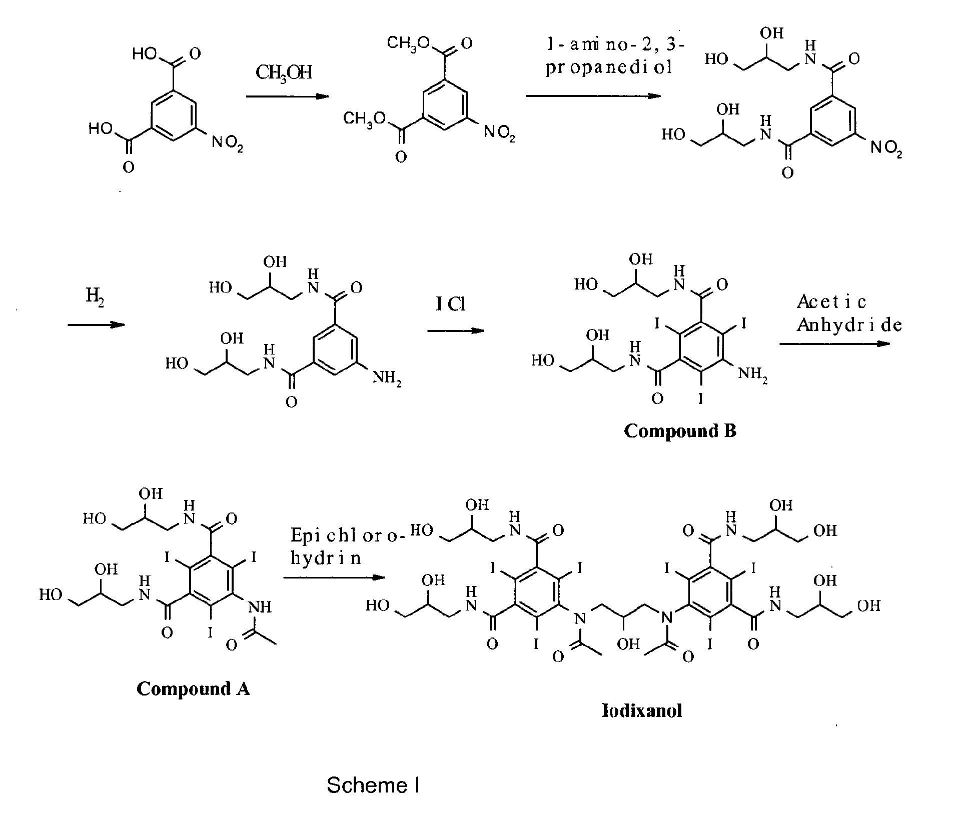

The reaction is usually carried out in the non-aqueous solvent 2-methoxyethanol and generally results in the conversion of 40 to 60% of Compound A to iodixanol. The product contains large amounts of impurities and is normally purified by crystallization. Too large amounts of impurities make the purification difficult and to achieve the desired purity, the crude iodixanol produced by the synthetic chemical process is crystallized twice. The process is time consuming and takes about 3 days for the first crystallization and about 2 days for the second one. Hence, the crystallisation process is very demanding in terms of time and equipment size, it will take several days to perform and is often a bottleneck in industrial scale processes.

It is hence a desire to identify alternative low-cost and easily accessible solvents that can be used in the dimerisation step and that fulfill the above-mentioned criteria.

GB 2331098 describes the synthesis of iodixanol using water as solvent. However, the process described also uses boric acid to reduce the amount of impurities. Boric acid is not environmentally friendly and the level of impurities is still high in the final product.

EP 108638 describes the synthesis of iodixanol using water as solvent. However the concentration of Compound A in water is low and the amount of impurities in the final product is high.

It has now surprisingly been found that water can be used as solvent in the dimerisation step of Compound A in an industrial scale and will fulfill the requirements listed above.

### SUMMARY OF THE INVENTION

The present invention provides a large scale dimerisation process of 5-acetamido-N,N'-bis(2,3-dihydroxypropyl)-2,4,6-triiodo-isophthalamide to iodixanol.

Thus, the invention provides a process for the dimerisation of 5-acetamido-N,N'-bis(2,3-dihydroxypropyl)-2,4,6-triiodo-isophthalamide in a temperature range of about 10 to about 20°C using water as solvent in a concentration of about 0.8 to about 3 ml solvent per g 5-acetamido-N,N'-bis(2,3-dihydroxypropyl)-2,4,6-triiodo-isophthalamide in the presence of about 0.2 to about 0.4 mole equivalents of epichlorohydrin relative to 5-acetamido-N,N'-bis(2,3-dihydroxypropyl)-2,4,6-triiodo-isophthalamide, and wherein the reaction solution has a pH value of about 11.5 to about 13.0.

The instant process uses a low cost solvent that is environmentally friendly and provides high enough yields and purity in the final product to make the manufacturing process of iodixanol economically feasible in an industrial scale.

### DETAILED DESCRIPTION OF THE INVENTION

Crude iodixanol is obtained from the processes known from the state of art, e.g. from the dimerisation process illustrated in Scheme I above. The dimerisation step itself may be carried out as described in EP 108638 and WO 98/23296, for example using epichlorohydrin as the dimerisation agent. The reaction is usually carried out in the non-aqueous solvent 2-methoxyethanol and generally results in the conversion of 40 to 60% of Compound A to iodixanol.

Up until now water has been suggested as a possible alternative solvent in the dimerisation step for preparing iodixanol, however no documentation has revealed that the use of water can be feasible in an industrial scale. Low concentrations of Compound A in water used in prior art give large process solution volumes with low yield per batch and are therefore not suitable in an industrial scale. Also, boric acid has been used in prior art in order to reduce the amount of impurities in the final product, but boric acid should be avoided for environmental reasons. Hence, other solvents, especially 2-methoxyethanol have been used in large scale production of iodixanol.

As explained above the dimerisation generally results in the conversion of 40 to 60% of Compound A. However, the product contains large amounts of impurities and needs to undergo costly work-up procedures, like for example multiple crystallizations.

The most important impurities in the reaction with regard to work-up consequences are the so-called backpeaks. This term refers to retention times in reversed phase HPLC, where the backpeaks have slightly longer retention times than iodixanol itself. Most of the backpeaks are either trimers or O-alkylated dimers. Two examples are given below:

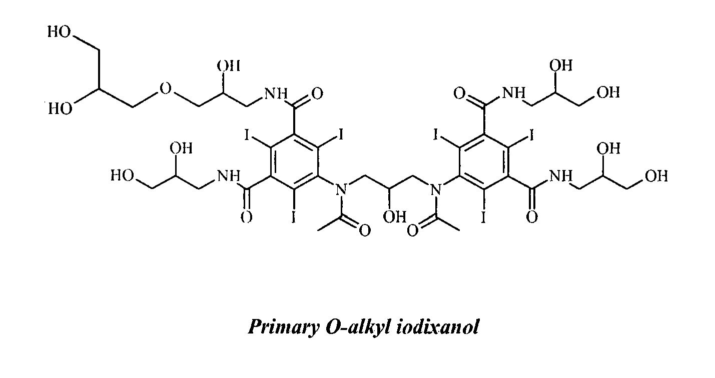

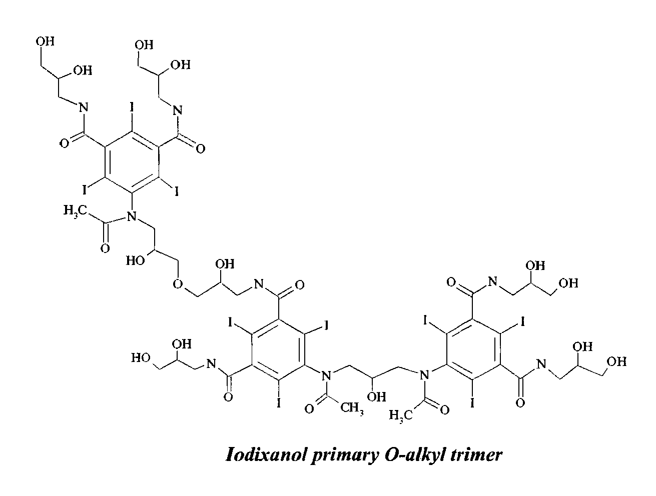

Other byproducts of importance are e.g. iohexol and N-acetyl cyclised iodixanol, whose structures are shown below. lohexol is fairly easy to remove in the subsequent crystallisation of iodixanol, even when present in several weight percent.

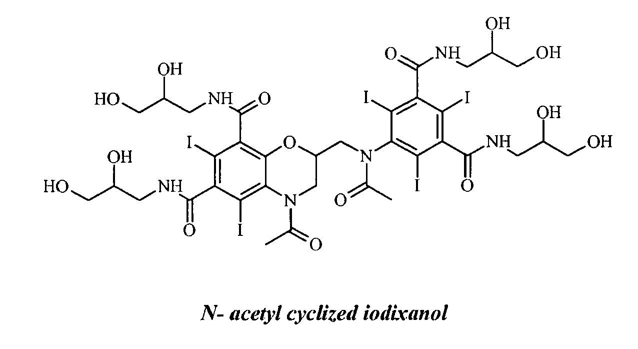

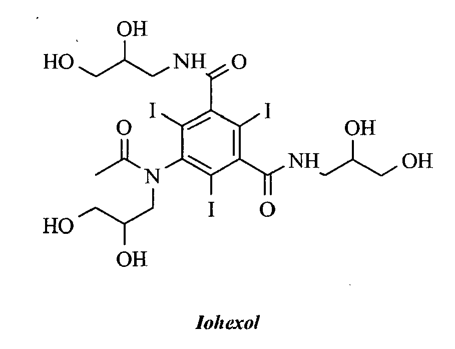

A typical selectivity required to be able to run an economically feasible work-up and obtain the required product quality is that the amount of backpeaks should not exceed 2% at 50-60% conversion of Compound A to iodixanol. At lower conversions the amount of backpeaks should be even lower, e.g. not more than about 1% at about 40% conversion.

It has now surprisingly been found that under specific conditions the dimerisation step can be carried out with water as solvent resulting in a product that meets the requirements in order to make the overall process of manufacturing iodixanol feasible. Most importantly, it has been found that by lowering the amount of epichlorohydrin added acceptable amounts of backpeaks in the final product can be achieved.

Additionally, increasing the concentration of Compond A in water in order to make the process feasible in an industrial scale would lead to an increase in backpeaks, creating difficulties in the crystallization step and a final product not pure enough to meet the set standards. Carrying out the dimerisation under reduced temperature, e.g. below room temperature, reduces the amount of backpeaks and makes it possible to carry out the dimerisation in higher concentrations.

Thus, the invention provides a process for the preparation of 1,3-bis(acetamido)-N,N'-bis[3,5-bis(2,3-dihydroxypropylaminocarbonyl)-2,4,6-triiodophenyl]-2-hydroxypropane by dimerisation of 5-acetamido-N,N'-bis(2,3-dihydroxypropyl)-2,4,6-triiodo-isophthalamide, the process being carried out under the following conditions:
i) at a temperature range of about 10 to about 20°C;
ii) using water as solvent in a concentration of about 0.8 to about 3 ml solvent per g 5-acetamido-N,N'-bis(2,3-dihydroxypropyl)-2,4,6-triiodo-isophthalamide;
iii) in the presence of about 0.2 to about 0.4 mole equivalents of epichlorohydrin relative to 5-acetamido-N,N'-bis(2,3-dihydroxypropyl)-2,4,6-triiodo-isophthalamide; and iv) wherein the reaction solution has a pH value of about 11.5 to about 13.0.

The process according to the present invention is carried out with a concentration of about 0.8 to about 3 ml solvent per g Compound A, more preferably below 2.0 and most preferably about 1.0.

The temperature during the dimerisation should be in the range of about 10 to about 20°C, but even more preferred is about 10 to about 15°C. The temperature can be constant throughout the dimerisation or varied within the specified range, preferably the temperature is lowered throughout the dimerisation.

The dimerisation agent used in the present invention is epichlorohydrin which is added in about 0.2 to about 0.4 mole equivalents, preferably about 0.23 to about 0.36.

The pH value in the reaction solution is about 11.5 to about 13.0, but even more preferably about 12.1 to about 11.7. The pH value can preferably be varied throughout the dimerisation having a higher value at the start of the dimerisation than at the end.

The base used to raise the pH value of the reaction solution can be any base suitable. Preferably the base is sodium hydroxide (NaOH) or potassium hydroxide (KOH), with sodium hydroxide being most preferred.

For further adjustment of the pH value of the reaction solution any suitable acid can be used, preferably concentrated hydrochloric acid (HCI).

The dimerisation step will be allowed to proceed for several hours with a preferred reaction time of 12 to 48 hours and particularly preferred from 24 to 48 hours. The reaction may be terminated by quenching with any acid, preferably hydrochloric acid. The reaction may be monitored, e.g. by HPLC, to determine the appropriate stage at which quenching should take place.

The invention is illustrated further by the following examples that are not to be construed as limiting the invention in scope to the specific procedures or products described in them.

### EXAMPLES

### EXAMPLE 1

Sodium hydroxide pellets (1.19 eq.) was dissolved in water (250 ml) and 5-acetamido-N,N'-bis(2,3-dihydroxypropyl)-2,4,6-triiodo-isophthalamide (Compound A) (50 g) was added. pH was adjusted with 2 M hydrochloric acid from 12.7 to 12.2 and the mixture was cooled to 20°C followed by addition of epichlorohydrin (0.27 eq) added in three portions over 70 minutes. After 48 hours an HPLC analysis showed the following composition: 43.5 % iodixanol, 0.8 % backpeaks and 5.1 % iohexol.

### EXAMPLE 2

Sodium hydroxide pellets (1,19 eq.) was dissolved in water (150 ml) and 5-acetamido-N,N'-bis(2,3-dihydroxypropyl)-2,4,6-triiodo-isophthalamide (Compound A) (50 g) was added. pH was adjusted with 2 M hydrochloric acid from 12,7 to 12,2 and the mixture was cooled to 20°C followed by addition of epichlorohydrin (0.27 eq) added in three portions over 70 minutes. After 24 hours an HPLC analysis showed the following composition: 45.6 % iodixanol, 1.4 % backpeaks and 4.6 % iohexol.

### EXAMPLE 3

Sodium hydroxide pellets (1,19 eq.) was dissolved in water (140 I) and 5-acetamido-N,N'-bis(2,3-dihydroxypropyl)-2,4,6-triiodo-isophthalamide (Compound A) (70 kg) was added. pH was adjusted with 2 M hydrochloric acid from 12,7 to 12,2 and the mixture was cooled to 15°C followed by addition of epichlorohydrin (0.27 eq) added in three portions over 70 minutes. After 16 hours an HPLC analysis showed the following composition: 38 % iodixanol, about 1 % backpeaks and < 4 % iohexol.

## Claims

1. Process for the preparation of 1,3-bis(acetamido)-N,N'-bis[3,5-bis(2,3-dihydroxypropylaminocarbonyl)-2,4,6-triiodophenyl]-2-hydroxypropane by dimerisation of 5-acetamido-N,N'-bis(2,3-dihydroxypropyl)-2,4,6-triiodoisophthalamide, the process being carried out under the following conditions:
i) at a temperature range of 10 to 20°C;
ii) using water as solvent in a concentration of 0.8 to 3 ml solvent per g 5-acetamido-N,N'-bis(2,3-dihydroxypropyl)-2,4,6-triiodo-isophthalamide;
iii) in the presence of 0.2 to 0.4 mole equivalents of epichlorohydrin relative to 5-acetamido-N,N'-bis(2,3-dihydroxypropyl)-2,4,6-triiodo-isophthalamide; and
iv) wherein the reaction solution has a pH value of 11.5 to 13.0.

## Patentansprüche

1. Verfahren zur Herstellung von 1,3-Bis(acetamido)-N,N'-bis[3,5-bis(2,3-dihydroxypropylaminocarbonyl)-2,4,6-triiodphenyl]-2-hydroxypropan durch Dimerisierung von 5-Acetamido-N,N'-bis(2,3-dihydroxypropyl)-2,4,6-triiodisophthalamid, wobei das Verfahren unter den folgenden Bedingungen ausgeführt wird:
i) in einem Temperaturbereich von 10 bis 20 °C;
ii) unter Verwendung von Wasser als Lösungsmittel in einer Konzentration von 0,8 bis 3 ml Lösungsmittel pro g 5-Acetamido-N,N'-bis(2,3-dihydroxypropyl)-2,4,6-triiod-isophthalamid;
iii) bei Vorhandensein von 0,2 bis 0,4 Mol-Äquivalenten Epichlorhydrin bezogen auf 5-Acetamido-N,N'-bis(2,3-dihydroxypropyl)-2,4,6-triiodisophthalamid; und
iv) wobei die Reaktionslösung einen pH-Wert von 11,5 bis 13,0 aufweist.

## Revendications

1. Procédé de préparation de 1,3-bis(acétamido)-N,N'-bis[3,5-bis(2,3-dihydroxypropylaminocarbonyl)-2,4,6-tri-iodophényl]-2-hydroxypropane par dimérisation de 5-acétamido-N,N'-bis(2,3-dihydroxypropyl)-2,4,6-triiodoisophthalamide, le procédé étant mis en oeuvre sous les conditions suivantes :
i) dans une plage de température de 10 à 20°C ;
ii) en utilisant de l'eau en tant que solvant à une concentration de 0,8 à 3 ml de solvant par g de 5-acétamido-N,N'-bis(2,3-dihydroxypropyl)-2,4,6-triiodo-isophthalamide ;
iii) en la présence de 0,2 à 0,4 mole équivalent d'épichlorohydrine par rapport à 5-acétamido-N,N'-bis(2,3-dihydroxypropyl)-2,4,6-triiodoisophthalamide ; et
iv) dans lequel la solution de réaction présente une valeur de pH de 11,5 à 13,0.
